Europäisches Patentamt

European Patent Office

Office européen des brevets

(19)

(11) Publication number: **0 174 026 B1**

# EUROPEAN PATENT SPECIFICATION

(12)

(45) Date of publication of patent specification: **19.02.92** (51) Int. Cl.[5]: **G01N 33/577**

(21) Application number: **85111223.5**

(22) Date of filing: **05.09.85**

(54) **Nonspecific-reaction inhibitor in an immunological assay with monoclonal antibody.**

(30) Priority: **05.09.84 JP 187170/84**

(43) Date of publication of application:
**12.03.86 Bulletin 86/11**

(45) Publication of the grant of the patent:
**19.02.92 Bulletin 92/08**

(84) Designated Contracting States:
**AT BE CH DE FR GB IT LI NL SE**

(56) References cited:
**EP-A- 0 010 932**
**EP-A- 0 051 096**
**GB-A- 2 098 730**

**CHEMICAL ABSTRACTS, vol. 66, no. 22, 29 May 1967, Columbus, OH (US); J.MUNOZ, p. 9677, no. 103581g**

**PATENT ABSTRACTS OF JAPAN, vol. 8, no. 204 (P-301)[1641], 18 September 1984**

(73) Proprietor: **Juridical Foundation The Chemo-Sero-Therapeutic Research Institute
668, Okubo Shimizu-machi
Kumamoto-shi Kumamoto-ken(JP)**

(72) Inventor: **Tsuji, Ichiroh
5-18-27, Wakaba
Kumamoto-shi Kumamoto-ken(JP)**
Inventor: **Unoki, Masanori
622-6, Shinchi Shimizu-machi
Kumamoto-shi Kumamoto-ken(JP)**
Inventor: **Aoyama, Noriaki
357, Yamamuro Shimizu-machi
Kumamoto-shi Kumamoto-ken(JP)**

(74) Representative: **Vossius & Partner
Siebertstrasse 4 P.O. Box 86 07 67
W-8000 München 86(DE)**

**Description**

This invention relates to a nonspecific-reaction inhibitor for use in an immunological assay with monoclonal antibodies.

It has recently been reported that monoclonal antibodies (MCAs) can be advantageously used in several immunological assay methods such as PHA (passive hemagglutination assay), RPHA (reversed passive hemagglutination assay), RIA (radioimmunoassay) and EIA (enzyme immunoassay) since they have high specificity against an antigen (the same type of antigen as the one with which the antibody is obtained) and produce good results.

It is known that MCAs, not only as a solid phase antibody but also as a labeled antibody, can be used to assay mainly HBs (hepatitis B virus surface) antigen. (Unexamined PCT Japanese Patent Publication No. 58 (1983) - 500300)

There is also known an immunoassay method in which fixed MCAs bind to a labeled antigen (Unexamined Japanese Patent Publication No. 58 (1983) - 7146) and an EIA method for determing TSH (thyroid-stimulating hormone) or other analogous hormones with MCAs (CLINICAL CHEMISTRY, Vol.28, No.9, pp.1862 - 1866, 1982).

Many other papers and patents also refer to the use of MCAs for immunological assays.

Generally when these immunological assay methods are carried out, there is used a monoclonal antibody (MCA) obtained from a hybridoma which was obtained by fusing spleen cells of a mouse immunized with the antigen to be determined with a mouse myeloma cell.

The RIA and EIA methods are two of the most sensitive immunological assay methods among those conventionally used.

These can be carried out either by the one-step method or by the two-step method.

In the two-step method, the MCA is adsorbed on a solid phase carrier such as polystyrene beads, reacted with the antigen to be determined, which is then reacted with a MCA labeled with a radio isotope or an enzyme. Then the value of the antigen is assayed by measuring the radioactivity or enzyme activity.

In another embodiment labeled MCA and the antigen to be determined, are first allowed to react and are then reacted with a MCA immobilized on a solid phase.

In the one-step method, a solid phase adsorbed MCA and a labeled MCA are mixed and reacted with the antigen and the assay is carried out by measuring the radioactivity or enzyme activity.

Alternatively, either the solid phase antibody or the labeled antibody is a MCA and the other one is a polyclonal antibody.

In the use of MCAs from mouse as a solid phase and/or labeled (conjugated) antibody in such an assay as described above, the presence of various substances other than the antigen to be assayed, may cause the inaccuracy of the assay. For example, the presence of anti-mouse immunoglobulin antibodies such as anti-mouse IgG and IgM in the samples causes an antigen negative sample to look as if it were a positive one. This is probably because the anti-mouse immunoglobulin antibodies react with solid phase MCAs and/or conjugated MCAs. Therefore, it is necessary to inhibit the nonspecific reaction in order to obtain exact data.

EP-A1 010932 discloses the use of an active fraction of mouse ascitic fluid as a reagent in an immunoassay which reagent has the ability to combine with antigen-antibody complexes but not with free antibodies or antigens.

The present inventors have studied an improved method for an immunological assay with MCAs, and have found that mouse serum, mouse ascites, rat serum or rat ascites serve as extremely effective nonspecific-reaction inhibitors. The present invention, therefore, is directed to the use of antibody-containing rat or mouse ascites fluid or serum for inhibiting non-specific reactions in immunological assays in which an antigen is to be quantified or detected in a sample with a mouse monoclonal antibody against the antigen. because the anti-mouse immunoglobulin antibodies in the sample react with the immunoglobulin in the mouse serum, mouse ascites, rat serum or rat ascites.

The MCA originating from mouse to be used in this invention is obtained according to the ordinary method by fusing spleen cells from an immunized mouse with myeloma cells to obtain a hybridoma and then cloning the hybridoma to obtain monoclonal antibodies.

The present invention will now be illustrated by experiments and examples, but should not be construed to be limited thereto.

Experiment 1

Spleen cells of a BALB/c mouse immunized against HBs were fused with mouse myeloma cells (P3-X63-Ag8-U1) by the ordinary method, and four samples thus obtained were measured by the EIA method using MCA against HBs antigen.

A two-step immunological assay was carried out as follows:

The sample (200 µl) was made to react with MCA fixed on polystyrene beads by the ordinary method at 37 °C for 2 hours in a test tube. The beads were then washed with 5 % Tween 20 - PBS three times and allowed to react with MCA (200 µl) labeled with horseradish peroxidase, at 37 °C for 2 hours. Next, the beads were washed with 0.5 % Tween® 20 - PBS three times and transferred to another test tube. To the test tube were added one solution containing 0.1 M citric acid and 0.2 M sodium phosphate (25 ml) and having a pH of 4.8 and another solution containing 5 % $H_2O_2$ (25 µl) -o-phenylenediamine (15 mg) (1.0 ml). After the addition, the mixture was allowed to stand in the dark for 1 hour at 37 °C, whereafter the reaction was stopped by the addition of 2N $H_2SO_4$ (1.0 ml). The absorbance of the solution was measured at 492 nm. The results are shown in Table 1.

## Table 1

| No. | Sample | Absorbance ($\lambda$=492 nm) |
|---|---|---|
| 1 | Both HBs antigen and antibody negative | 0.030 |
| 2 | HBs antigen negative | 0.581 |
| 3 | HBs Ag 10 ng/ml | 0.586 |
| 4 | HBs Ag 100 ng/ml | 2.537 |

Note: No. 1 and 2 were confirmed negative in advance by RIA or RPHA .

The results prove that a nonspecific reaction took place in sample No.2, which is HBs antigen negative, since it showed color development corresponding to the sample of HBs Ag 10 ng/ml.

Experiment 2

Sample No. 2, which revealed a nonspecific reaction in Experiment 1, was examined regarding the nonspecific-reaction inhibiting effects of a number of additives (1 v/v % or 1 w/w %).

The effects were evaluated by the use of the one-step EIA method and the two-step EIA method.

In the one-step EIA, the measurement was carried out under the same conditions as in Experiment 1 except that the sample was prepared by mixing the polystyrene beads fixed with MCA and the labeled MCA all at once.

## Table 2

### ADSORBENT EFFECT OF ADDITIONS ON NONSPECIFIC REACTION

| No. | Additive | One-step method | Two-step method |
|---|---|---|---|
| 1 | none | 0.030 | 0.029 |
| 2 | none | 0.589 | 0.576 |
| 5 | mouse serum | 0.029 | 0.028 |
| 6 | mouse ascites | 0.030 | 0.030 |
| 7 | mouse ascites (containing anti-cholera MCA) | 0.031 | 0.029 |
| 8 | mouse ascites (containing anti-rabies MCA) | 0.030 | 0.031 |
| 9 | mouse ascites (containing anti-Japanese encephalitis MCA) | 0.029 | 0.030 |
| 10 | mouse ascites (containing anti-herpes MCA) | 0.030 | 0.028 |
| 11 | mouse kidney | 0.536 | 0.534 |
| 12 | mouse liver | 0.536 | 0.534 |
| 13 | rat serum | 0.134 | 0.129 |
| 14 | rat ascites | 0.158 | 0.149 |
| 15 | hamster serum | 0.233 | 0.223 |

-to be continued-

4

Table 2 (continued)

| No. | Additive | One-step method | Two-step method |
|-----|----------|-----------------|-----------------|
| 16 | sheep serum | 0.281 | 0.293 |
| 17 | horse serum | 0.284 | 0.290 |
| 18 | guinea pig serum | 0.294 | 0.291 |
| 19 | bovine serum | 0.269 | 0.258 |
| 20 | fetal bovine serum | 0.294 | 0.289 |
| 21 | swine serum | 0.336 | 0.351 |
| 22 | rabbit serum | 0.345 | 0.348 |
| 23 | dog serum | 0.467 | 0.478 |
| 24 | avian serum | 0.506 | 0.518 |
| 25 | saline | 0.591 | 0.586 |
| 26 | PBS | 0.588 | 0.587 |
| 27 | gelatin | 0.578 | 0.593 |
| 28 | aluminum hydroxide | 0.593 | 0.588 |
| 29 | calcium phosphite | 0.598 | 0.586 |

Note:  (1) No. 1 and 2 correspond  to  those in Experiment 1

(2) No. 5 to 29 correspond  to  No. 2 in Experiment

1, but are numbered separately because of different

additions.

As shown in Table 2, mouse serum and mouse ascites have remarkable effects on inhibition (regardless of whether they contain MCA or not), while rat serum and rat ascites (No. 13 and 14) are also suitable for use as nonspecific-reaction inhibitors since they exhibit good effects (more than 80 %).

Among the other additives, although all of the animal sera exhibit some effect, none is effective enough for practical use.

Example 1

The inhibiting effects of mouse serum, mouse ascites, and rat serum were further examined with the same kind of random samples (sera), which had been in advance judged HBs antigen negative by a confirmative test.

After the addition of the inhibitor to the sample (1 v/v %), the solution was determined by the one-step EIA method.

For comparison, samples with and without an addition of saline were also measured by the method.

## Table 3

INHIBITING EFFECT BY NEGATIVE SERA (Absorbance $\lambda$=492 nm)

| No. | No Addition | Saline | mouse serum | mouse ascites | rat serum |
|-----|-------------|--------|-------------|---------------|-----------|
| 1 | 0.030 | 0.031 | 0.030 | 0.030 | 0.029 |
| 30 | 0.145 | 0.135 | 0.029 | 0.031 | 0.032 |
| 31 | 0.029 | 0.030 | 0.030 | 0.030 | 0.031 |
| 32 | 0.535 | 0.529 | 0.031 | 0.031 | 0.083 |
| 33 | 0.628 | 0.635 | 0.029 | 0.030 | 0.091 |
| 34 | 0.139 | 0.143 | 0.028 | 0.030 | 0.030 |
| 35 | 0.029 | 0.029 | 0.030 | 0.028 | 0.031 |
| 36 | 0.030 | 0.031 | 0.028 | 0.029 | 0.029 |
| 37 | 0.198 | 0.205 | 0.030 | 0.028 | 0.030 |
| 38 | 0.249 | 0.245 | 0.030 | 0.031 | 0.032 |
| 39 | 0.352 | 0.348 | 0.029 | 0.030 | 0.041 |
| 40 | 0.635 | 0.631 | 0.592 | 0.587 | 0.618 |

Note: (1) Sample No. 1 corresponds to that in Exp.1

(2) Sample No. 40 was HBs antigen positive (HBs antigen 10 ng/ml).It is deliberately included for comparison.

The effect of the nonspecific-reaction inhibitors of this invention can be seen from the results shown in Table 3, as follows:

1. The inhibitors of this invention have a very good effect on the nonspecific reaction regardless of how strong or weak it is. (As far as mouse serum and mouse ascites are concerned, the effect is nearly perfect.)

2. It is evident that the inhibitors have no effect on the assay of a sample not exhibiting nonspecific reaction.

3. Even in a sample of positive serum (No. 40), adding these inhibitors has no effect on the judgment.

The effect of varying the amount added of the nonspecific-reaction inhibitors was investigated.

To samples identical with No. 2 in Experiment 1 were added mouse serum or mouse ascites at various ratios, whereafter the samples were determined by the one-step EIA method. The absorbances at 492 nm are shown in Table 4.

Table 4

| Amount added (v/v%) | mouse serum | mouse ascites |
|---|---|---|
| 0 | 0.594 | 0.594 |
| 0.1 | 0.062 | 0.135 |
| 0.5 | 0.032 | 0.035 |
| 1.0 | 0.029 | 0.030 |
| 5.0 | 0.030 | 0.030 |

The results suggest that sufficient effect is obtained by adding at least 0.1 96 of the nonspecific-reaction inhibitor, although addition of from 0.5 to 1.0 v/v% gives even better results.

Inhibiting effects do not change if more of the nonspecific inhibitor is added.

Example 2

Spleen cells of a BALB/c mouse immunized with TSH were fused with mouse myeloma cells (P3-X63-Ag8-U1) by the ordinary method, and MCA against TSH was prepared by cloning.

With this MCA, a nonspecific-reaction test by the EIA method was carried out.

TSH negative serum was prepared by anti-TSH antibody (rabbit serum) conjugated CNBr-Sepharose® CL-4B affinity chromatography and was confirmed with a RIA kit.

The results are shown in Tables 5 and 6.

Table 5

| No. | Sample | Absorbance ($\lambda = 492$ nm) Two-step method |
|---|---|---|
| 41 | TSH negative | 0.021 |
| 42 | TSH negative | 0.480 |
| 43 | TSH 2.5 u/ml | 0.625 |
| 44 | TSH 10.0 u/ml | 0.362 |

## Table 6

| No. | Inhibitor | Absorbance (λ=492 nm) (Two-step method) |
|---|---|---|
| 41 | none | 0.018 |
| 42 | none | 0.429 |
| 45 | mouse serum | 0.020 |
| 46 | mouse ascites | 0.018 |
| 47 | rat serum | 0.107 |
| 48 | rat ascites | 0.123 |
| 49 | hamster serum | 0.354 |
| 50 | rabbit serum | 0.413 |
| 51 | gelatin | 0.597 |

Note: No. 45 to 51 correspond to No. 42, but are numbered separately because of different additions.

These results show that the nonspecific-reaction inhibitors of this invention are effective in an assay with MCA against TSH. Thus it is suggested that these inhibitors will also produce a good effect on the assay when other antigens and the respective MCAs are used according to the principle of this invention.

Generally, the nonspecific-reaction inhibitors can be used or supplied in the following ways, but according to the principle of this invention it is obviously also possible to apply the inhibitors in various other ways.

1. Addition to the determination system before and during measurement.

2. Addition to the sample and inhibited nonspecific-reaction substance before carrying out the assay.

3. In case of the solid phase method, it is added to the conjugate (liquid) in advance and lyophylized until use, if necessary.

4. Addition to the conjugated diluents or to the solution beforehand (in case of a lyophilized sample).

## Claims

1. Use of antibody-containing rat or mouse ascites fluid or serum for inhibiting non-specific reactions in immunological assays in which an antigen is to be quantified or detected in a sample with a mouse monoclonal antibody against the antigen.

2. Use according to claim 1, wherein the mouse antibodies are monoclonal antibodies contained in mouse ascites.

3. Use according to claim 1 wherein rat ascites containing rat antibodies is used.

4. Use according to claim 1, wherein the contained mouse or rat antibodies are polyclonal antibodies.

5. Use according to any one of claims 1 to 4 wherein the ascites fluid or serum is added in an amount of 0.1 to 1.0 v/v% of the sample to be determined in the assay.

8

6. Use according to claim 5, wherein the ascites fluid or serum is added to the diluent used for diluting the sample in the immunological assay.

7. Use according to claim 5, wherein the ascites fluid or serum is contained in a solution of labeled antibody or in a lyophylized labeled antibody for enzyme immunoassay or radio immunoassay.

**Revendications**

1. Utilisation de sérum ou de liquide ascitique de souris ou de rat contenant des anticorps en vue de l'inhibition de réactions aspécifiques au cours d'essais ou titrages immunologiques dans lesquels il faut déceler ou quantifier un antigène dans un échantillon avec un anticorps monoclonal de souris vis-à-vis de l'antigène.

2. Utilisation suivant la revendication 1, caractérisée en ce que les anticorps de souris sont des anticorps monoclonaux contenus dans le liquide ascitique de souris.

3. Utilisation suivant la revendication 1, caractérisée en ce que l'on utilise du liquide ascitique de rat contenant des anticorps de rat.

4. Utilisation suivant la revendication 1, caractérisée en ce que les anticorps de rat ou de souris contenus sont des anticorps polyclonaux.

5. Utilisation suivant l'une quelconque des revendications 1 à 4, caractérisée en ce que l'on ajoute le sérum ou le liquide ascitique en une proportion de 0,1 à 1,0% v/v de l'échantillon à déterminer au cours de l'essai ou titrage.

6. Utilisation suivant la revendication 5, caractérisée en ce que l'on ajoute le sérum ou le liquide ascitique au diluant employé pour diluer l'échantillon au cours de l'essai ou titrage immunologique.

7. Utilisation suivant la revendication 5, caractérisée en ce que le sérum ou le liquide ascitique est contenu dans une solution d'anticorps marqué, ou dans un anticorps marqué, lyophilisé, pour radio-immunotitrage ou -essai ou immunotitrage ou -essai enzymatique.

**Patentansprüche**

1. Verwendung einer Ascitesflüssigkeit oder eines Serums von Ratte oder Maus, enthaltend Antikörper, zur Inhibition nichtspezifischer Reaktionen in immunologischen Tests, bei denen ein Antigen in einer Probe mit einem monoclonalen Maus-Antikörper gegen das Antigen quantifiziert oder nachgewiesen werden soll.

2. Verwendung nach Anspruch 1, wobei die Maus-Antikörper monoclonale Antikörper sind, die in Maus-Ascites enthalten sind.

3. Verwendung nach Anspruch 1, wobei Ratten-Ascites, enthaltend Ratten-Antikörper, verwendet wird.

4. Verwendung nach Anspruch 1, wobei die enthaltenen Maus- oder Ratten-Antikörper polyclonale Antikörper sind.

5. Verwendung nach einem der Ansprüche 1 bis 4, wobei die Ascitesflüssigkeit oder das Serum in einer Menge von 0,1 bis 1,0 Volumenprozent der im Test zu bestimmenden Probe zugesetzt wird.

6. Verwendung nach Anspruch 5, wobei die Ascitesflüssigkeit oder das Serum zum Verdünnungsmittel zugesetzt wird, das zur Verdünnung der Probe in dem immunologischen Test verwendet wird.

7. Verwendung nach Anspruch 5, wobei die Ascitesflüssigkeit oder das Serum in einer Lösung markierten Antikörpers oder in einem lyophilisierten markierten Antikörper für den Enzymimmuntest oder den Radioimmuntest enthalten ist.